# EUROPEAN PATENT APPLICATION

(11) **EP 1 577 674 A1**
(43) Date of publication of application: **21.09.2005**
(21) Application number: 04006111.1
(22) Date of filing: 15.03.2004
(51) Int. Cl.: G01N 33/76, G01N 33/78

(54) **Recombinant human tsh in the diagnosis of central hypothyroidism**

(71) Applicant: Sahltech I Göteborg AB, 413 45 Göteborg (SE)
(72) Inventor: Nyström, Ernst, Dpt. of Internal Medicine, 413 45 Göteborg (SE); Johannsson, Gudmundur, 421 63 Västra Frölunda (SE); Filipsson, Helena, 413 45 Göteborg (SE)
(74) Representative: Becker Kurig Straus

(57) **Abstract**

The present invention relates to the use of recombinant human TSH for diagnosing hypothyroidism. Specifically, the present invention pertains to the use of recombinant human TSH for diagnosing central hypothyroidism.

## Description

The present invention relates to a method for diagnosing hypothyroidism. In particular, the present invention pertains to the use of recombinant human TSH (rhTSH) for diagnosis of central hypothyroidism.

The thyroid is an endocrine gland comprised of follicle cells and non-follicular or C cells. The follicle cells produce thyroid hormones, while the C cells produce calcitonin which appears to influence calcium metabolism.

The thyroid gland depends for physiological functioning on thyrotropin, a product of the pituitary gland secreted in response to thyrotropin releasing hormone (TRH) produced by hypothalamic neurons. Thyrotropin stimulates the thyroid gland for the synthesis and secretion of thyroid hormones, thyroxine (T4) and triiodothyronin (T3), both of which influence the hypothalamus and the pituitary gland by a negative feedback mechanism. The reverse is true in case of low thyroxine and triiodothyronin levels in the blood regulating TSH levels up.

Thyrotropin stimulates processes in the thyroid gland in that the thyroid cells produce thyroglobulin, a tyrosine rich protein. Iodide actively transported into the cells is incorporated into the tyrosine residues and the tyrosyl radicals are then condensed to thyroxine. Thyroglobulin is degraded and thyroxine and triiodothyronin are released into the blood stream where they are bound to plasma proteins. The biological active form of the thyroid hormones is triiodothyronin (T3), which mainly results from extra-thyroidal de-iodination of thyroxine. The action of thyroid hormone is mainly directed to controlling the metabolic rate e.g. by increasing energy production and oxygen consumption by most tissues.

Among humans, thyroid disorders are quite common, with about two hundred million people being afflicted worldwide. Disorders of the thyroid include autoimmune disorders (e.g. Graves' disease, chronic lymfocytic thyroiditis), thyroiditis (inflammation or, rarely, infection of the thyroid), and cancer, all of which conditions may result in hypothyroidism or hyperthyroidism. An enlarged thyroid (goiter) may be euthyroid, or a symptom of either hyperthyroidism (thyrotoxicosis) or hypothyroidism.

Most cases of hyperthyroidism are believed to be due to the action of thyroid stimulating antibodies upon the thyroid as a whole (e.g. Graves' disease, diffuse toxic goiter). Causes of hyperthyroidism besides Graves' disease include toxic multinodular goiter, toxic adenoma, subacute viral thyroiditis, postpartum thyroiditis, thyroid, gonadal and pituitary tumors and excess pituitary, TSH.

In hypothyroidism, the thyroid gland is characterised by its hypofunction. In primary hypothyroidism, the thyroid gland becomes unable to synthesise hormones, most commonly due to an autoimmune condition or iodine deficiency. In central hypothyroidism, the hypothyroid condition is caused by a decrease in secretion of biologically active thyrotropin, due to a failure at the pituitary or hypothalamic level.

In theory, such a condition might be considered to be associated with low levels of serum thyrotropin and serum thyroid hormone. However, in the absence of hypothalamic stimulation by the thyrotropin releasing factor, TRH, the pituitary gland will secrete a thyrotropin in a low glycosylated form exhibiting a low bio-activity. This form of thyrotropin has a longer half-life than the normally produced thyrotropin, and therefore, in most cases, produces normal serum levels. It should in this context be noted that all immunoassays today in use for the determination of thyrotropin will determine this substance irrespective of its bioactivity.

Recognising the difficulty of diagnosing central hypothyroidism no satisfactory means presently available, with which central hypothyroidism may quickly and reliably be diagnosed.

Accordingly, a problem of the present invention is to provide a means for diagnosing central hypothyroidism.

In the course of the extensive studies leading to the present invention it has now surprisingly been found that administration of rhTSH to individuals suffering from central hypothyroidism results in an increase of thyroglobulin level as in healthy individuals but that in contrast to healthy controls thyroid hormone levels stay constant.

Therefore, the problem has been solved by providing a method for diagnosing central hypothyroidism, which comprises the step of determining the amount of thyroglobulin and thyroid hormone in a sample obtained from a patient, to which rhTSH has been administered, wherein an increase in thyroglobulin level and at the same time a constant thyroid hormone level is indicative that the individual has developed or suffers from central hypothyroidism.

In the figures,
Fig. 1 thyroglobulin (Tg) concentrations of CH, non-CH and CTL after rhTSH (0.1 mg and 0.9 mg) administration are shown.
Fig. 2 free T4 (FT4) concentrations of CH, non-CH and CTL after rhTSH (0.1 mg and 0.9 mg) administration are shown.
Fig. 3 total T4 (TT4) concentrations of CH, non-CH and CTL after rhTSH (0.1 mg and 0.9 mg) administration are shown.
Fig. 4 free T3 (FT3) concentrations of CH, non-CH and CTL after rhTSH (0.1 mg and 0.9 mg) administration are shown.
Fig. 5 total T3 (TT3) concentrations of CH, non-CH and CTL after rhTSH (0.1mg and 0.9 mg) administration are shown.

Thyrotropin is acknowledged to be the major regulator of the thyroid gland. The gene sequence has been elucidated and published (Wondisford FE et al. Mol Endocrinol. 1988; Jan 2(1):32-9: Cloning of the human thyreotropin beta-subunit gene and transient expression of biologically active human thyrotropin after gene transfection.), and the nucleotide sequence has been engineered and introduced into various expression systems (e.g. E. coli, yeast, baculovirus, CHO or mammalian cells) for producing recombinant human thyrotropin (rhTSH).

In the present studies eventually leading to the present invention, it has been found that the administration of rhTSH results in levels of thyroglobulin (Tg) and thyroid hormone, that allows a clear discrimination between patients with well defined CH as compared to controls. This has been shown by a study involving 6 patients with well-established central hypothyroidism (CH) associated with multiple pituitary hormone deficiency, 6 non-CH with pituitary disease but with intact secretion of TSH and 6 healthy matched controls (CTL). It has been found that 48 hours after administration of rhTSH thyroglobulin significantly increased in all three groups studied. In contrast, patients with CH showed no or a blunted increase in serum thyroid hormone levels, whereas in non-CH and CTL there occurred a similar increase in total and free thyroid hormone levels with no overlap with the patients previously verified CH.

All three categories investigated (CH, non-CH and controls) responded with an increase in serum thyroglobulin after administration of rhTSH. The increase in serum thyroglobulin appears to be more pronounced in CH and non-CH patients as compared to control subjects. An increase in serum thyroid hormones with a peak after 24 hours was seen in non-CH patients and controls, i.e. individuals with intact pituitary-thyroid axis. In contrast, CH patients showed no or a very blunted response.

Without wishing to be bound by any theory it is presently believed that these findings may in case be explained by the absence of TSH in the CH subjects. Furthermore, if the CH subjects still have a residual capacity to produce TSH, albeit in inadequate amount, this production might be suppressed by the chronic administration of thyroid hormones in these individuals.

The response in thyroglobulin was expected to be less pronounced in CH patients as compared to non-CH patients and controls because of the long-term absence of TSH stimulation of the thyroid. The increase in thyroglobulin that tended to be even greater in response to rhTSH stimulation was therefore entirelly unexpected.

A striking finding is the essential lack of a response to rhTSH supply in thyroid hormone secretion in CH patients as compared to non-CH patients and controls. Again, without wishing to be bound by any theory, one reason for the low release of thyroid hormones in CH patients after rhTSH adminstration might be a decrease in the iodine level in patients the tyroid gland of whom is not stimulated by TSH. Furthermore, the transport of thyroglobulin necessary for thyroid hormone synthesis might also have been affected.

Accordingly, rhTSH will be administered to an individual in an amount sufficient to elicit the desired response, which amount normally is in a range of from about 1 µg/kg to about 15 µg/kg body weight, preferably about 1.4 to 12.9 µg/kg, more preferably about 5 to 10 µg/kg. TSH may be adminstered as such or in form of its pharmaceutically acceptable salts. The route of administration may be any route which will effectively transport the active agent to the thyroid gland. Routes such as oral, nasal, pulmonary, or parenteral (subcutaneous, intramuscular, intravenous) may be suitable, while an intramuscular route may be preferred.

At basal and after a period of 24 to 48 hours a sample is taken from the individual which is subjected to the assay. As the sample, blood as such or serum may be used.

The level of thyroglobulin is determined by immunoflourometric method (Delphia, Wallac Sweden AB) wherein an increase of about doubled is considered to represent an increase that may be used for the present diagnosis.

On the other hand a level of thyroid hormone is considered to have stayed essentially the same if, the change from basal levels is less than 25%, preferably, less than 20 %, more preferably less than 15 % , even more preferred less than 10 %.

In principle, the the level of thyroid hormone may be determined by any method known in the art, e.g. by an immunochemoluminometric method (Architect, Abbott Scandinavia AB).

The present invention, therefore, provides for the use of recombinant human TSH (rhTSH) or a salt thereof for diagnosing/identifying central hypothyroidism in that the said polypeptide is administered to an individual and the effect of this administration on the level of two hormones in the individual is determined. If the patient shows an increased level of thyroglobulin and at the same time a level of thyroid hormone that is essentially unchanged, this is indicative of the disease condition assayed for.

The present invention also comprises a kit, which contains rhTSH or a pharmaceutically acceptable salt and means to determine the level of thyroglobulin and thyroid hormone. Optionally, the kit may also comprise buffers and reagents used for the assay reactions. Both the means for detecting thyroglobulin and for determining the level of thyroid hormone comprises patients of suspicion for pituitary insufficiency.

The following examples illustrate the invention without limiting it thereto.

### Examples:

In this study we followed the scheme commonly used for temporary interruption of levothyroxine administration to thyroidectomized thyroid cancer patients before radioiodine administration, i.e. three weeks with triiodothyronine followed by one week with no thyroid hormones. Basal thyroid hormone levels before administration of rhTSH were significantly lower in CH patients compared to non-CH patients and controls. As expected, serum TSH levels were low. Thus, in this setting, determination of thyroid hormones and TSH will discriminate CH patients. However, newly diagnosed patients with CH often exhibit normal or subnormal (free) thyroxine levels before initiation of thyroxine treatment. Furthermore, the normal reference range of FT4 is wide but the intraindividual variation is small, ±25% [20]. Thus, a patient with CH may present thyroxine levels within the lower part of the reference interval [21, 22]. The very low thyroid hormone levels found in our study of CH patients after interruption of levothyroxine substitution may mainly be due to a long time and total absence of effect of TSH on the thyroid gland due to long-standing hypopituitarism and levothyroxine suppression of thyrotropin from the pituitary. The effect of a longstanding thyroxine treatment on the paracrine effect of thyreostimulin [23] may also affect the basal thyroid levels after interruption of levothyroxine.

### Patients

Twelve patients with well-defined pituitary disease and multiple anterior pituitary hormone insufficiencies were recruited from the outpatient clinic of the Department of Endocrinology, Sahlgrenska University Hospital. In previous endocrinological evaluations six were diagnosed as having thyrotropin insufficiency and were classified as central hypothyroidism (CH). Six were found to have non-affected thyrotropin secretion, and were classified as non-central hypothyroidism (non-CH). Six healthy controls were also included in the study. The groups were matched for age, sex and BMI. Patients and controls with presence of antibodies against thyroperoxidase and patients with cardiac disease and treatment with anti-epileptic, antipsychotic and anticoagulation drugs were not eligible for the study.

Patients and controls were informed in writing about the study protocol and included after informed consent. The study protocol was approved by the Ethical Committee of Göteborg University and the Swedish Medical Product Agency Uppsala, Sweden.

### Study design

All 18 individuals were randomly assigned to either 0,1 or 0,9 mg of recombinant human TSH (rhTSH; Thyrogen®, Genzyme, USA) one week apart. The rhTSH was given as an intramuscular injection in the gluteal area at 09 AM.

Before the administration of rhTSH, the six patients with known central hypothyroidism and consequently ongoing levothyroxine substitution, were switched to triiodothyronin treatment (Liothyronin®, Nycomed AB, Sweden); 20 µg 3 times a day. After 3 weeks, triiodothyronin was discontinued for one week before administration of recombinant TSH as described above.

Before the start of the study, and before the second injection of recombinant TSH, the patients underwent an electrocardiographic control as well as a routine biochemical control (haemoglobin, and serum sodium, potassium, creatinine, calcium and liver function tests). Side effects were registered at each sampling occasion.

### Biochemical methods

Blood was sampled with the individual in a recumbent position 45 minutes before administration of recombinant TSH, at the time of injection and 2, 3½, 7, 24, 48 and 72 hours after. At each sampling occasion serum concentrations of TSH, thyroid hormones and thyroglobulin was determined.

*TSH* was analysed by an immunochemoluminometric method (Architect, Abbot Scandinavia AB). Reference interval 0,20-4,0 mIU/L. Interindividual coefficiency of variation(CV) 3%. *FT4*:: immunochemoluminometric method (Architect, Abbot Scandinavia AB). Reference interval 10-22 pmol/l. Interindividual CV 6% at low levels and 5% at high levels. *TT4:* immunochemoluminometric method (Architect, Abbot Scandinavia AB). Reference interval 56-147 nmol/L. Interindividual CV 5%. *FT3:* immunometric method (Architect, Abbot Scandinavia AB). Reference interval 2,6-5,7 pmol/l. Interindividual CV 9% at low levels and 4% at high levels. *TT3 :* immunometric method (Architect, Abbot Scandinavia AB). Reference interval 0,90-2,4 mmol/L. Interindividual CV 7% at low levels and 3% at high levels. *Thyroglobulin:* immunoflourometric method (Delphia, Wallac Sweden AB) with a detection limit of 0,20 µg/L. Reference interval 2-20 µg/L. Interindividual CV 3% at low levels, 8% at medium and 4% at high levels. *Anti thyroglobulin antibodies (TgAb)::* luminescence immunoassay (Lumitest, Brahms, Henningsdorf, Germany), analytical sensivity of 8,2 U/ml. Reference interval < 60 U/ml. Interindividual CV 9%. *Antithyroperoxidase antibodies (TPOAb)* :luminescensimmuno assay (Berilux 400, BRAHMS Diagnostica). Reference interval <60 kU/L. Interindividual CV in the reference range: 11 %.

All specimens were analysed in the same batch with the exception of thyroglobulin.

### Statistical methods

Descriptive data are presented as mean ± standard error of the mean (SEM). The overall within-group treatment effect in each trial was determined using Friedman's ANOVA for repeated measurements. Analysis of the effect of treatment was performed using Wilcoxon's matched pairs, signed-rank sum test. Analysis of between study levels was performed using the Mann-Whitney U-test. A statistical significance was considered finding a p-value <0,05.

### Results

All 18 included subjects fulfilled the study protocol. Two patients with CH and triiodothyronine substitution during four weeks before the administration of rhTSH experienced palpitations, and were therefore instructed to reduce triiodothyronine to reduce the dose by half. Some participants experienced slight side effects the week following the rhTSH administration (see Table 1) and fatigue being frequently reported event in the CH group (with discontinued thyroid hormone substitution). One patient showed pronounced tiredness, nausea, headache, muscle and back pain, symptoms relieved when the hydrocortisone replacement dose was increased. One patient experienced a brief period of chest pain, which after cardiac evaluation was classified as not being related ischemic heart disease.

**Table 1**

| Laboratory findings at baseline and after 48 hours after administration of 0.9 mg rTSH in patients with pituitary insufficiency and central hypothyroidism (CH), pituitary insufficiency and intact TSH secretion (nonCH), and controls. ' | | | | | | |
|---|---|---|---|---|---|---|
| **Analyte** | **CH Baseline** | **CH 48h** | **NonCH Baseline** | **NonCH 48h** | **Control Baseline** | **Control 48h** |
| Thyroglobulin µg/L | 13.0+/-5.5 | 32.9+/-13.2 | 8.2+/-2.1 | 77.8+/-12.5 | 3.0+/-0.7 | 47.0+/-9.5 |
| TSH mIE/L | 3.2+/-3.4 | 24.2+/-5.1 | 1.4+/-0.8 | 22.2+/-10.5 | 1.1+/-0.6 | 14.3+/-6.0 |
| FT4 pmol/L | 5.2+/-0.0 | 6.3+/-0.6 | 12.2+/-1.1 | 26.5+/-3.1 | 10.7+/-0.6 | 30.7+/-2.8 |
| T4 nmol/L | 26.1+/-3.9 | 37.1+/-7.8 | 77.3+/-6.6 | 163+/-18.0 | 60.8+/-7.2 | 160+/-13.7 |
| FT3 pmol/L | 2.5+/-0.5 | 3.5+/-0.7 | 4.2+/-2.0 | 9.6+/-0.8 | 3.8+/-0.3 | 10.3+/-1.0 |
| T3 nmol/L | 0.9+/-0.2 | 1.3+/-0.3 | 1.4+/-0.1 | 3.2+/-0.2 | 1.2+/-0.2 | 3.2+/-0.2 |

### Thyroglobulin (Tg)

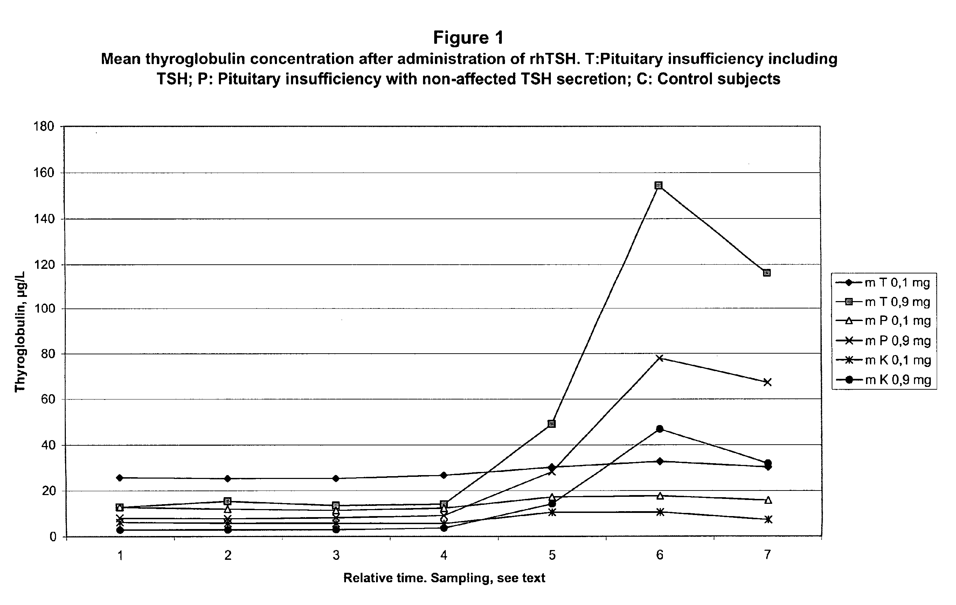

Tg concentrations are shown in Figure 1. At baseline, CH and non-CH have a higher level of Tg compared to CTL (p<0,05) (see Table 1) In all three groups a significant (p<0.05) increase in Tg was seen with a peak after 48 hours, which was greater after administration of the large dose of rhTSH (0.9mg).

### T4

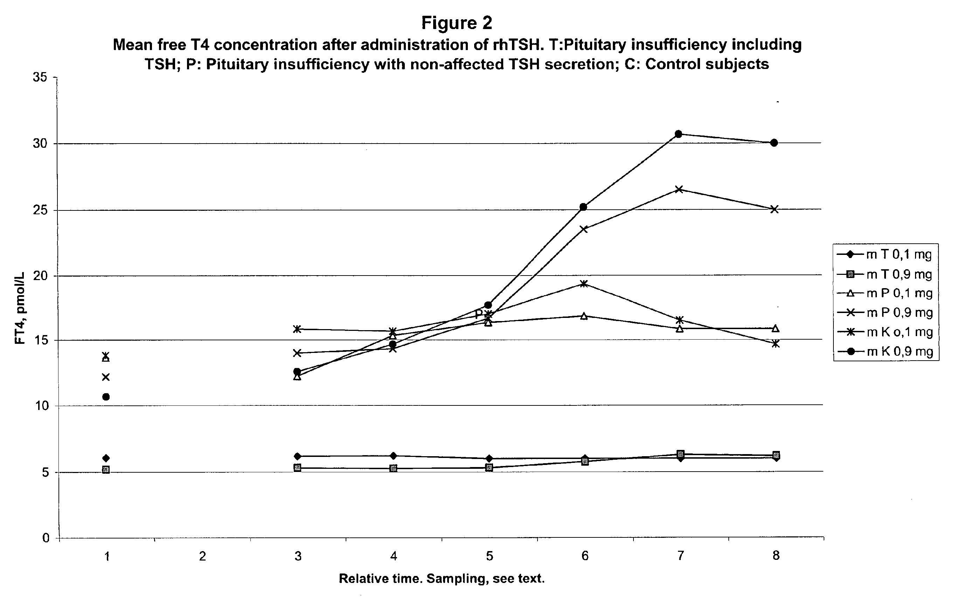

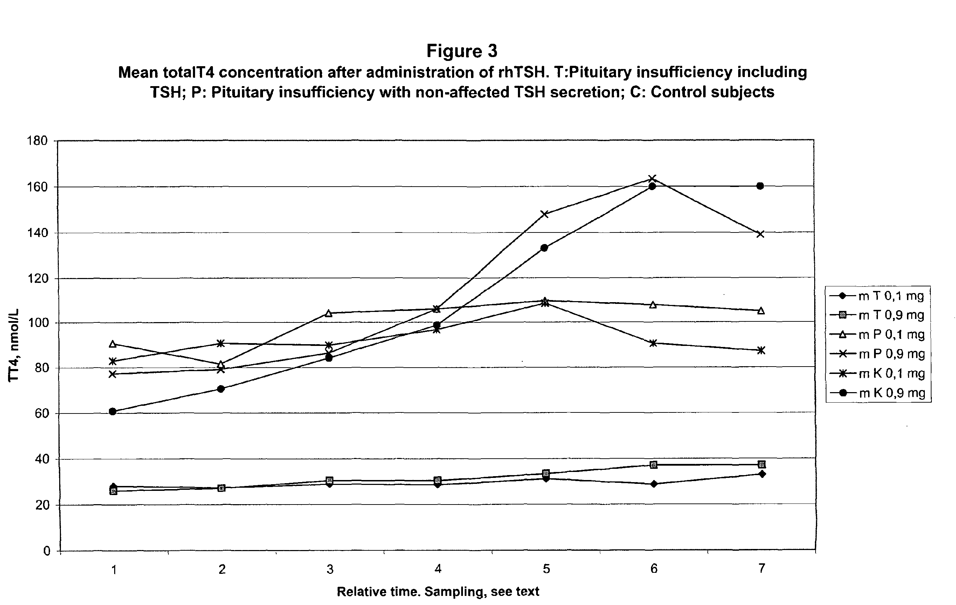

FT4 concentrations are shown in Figure 2. As shown in Table 1, CH patients had lower baseline FT4 values compared to controls (p<0,0001),whereas non-CH patients did not differ from controls. After receiving 0.1mg rhTSH no increase in FT4 was recorded in CH patients, and only a blunt response after the higher dose (0.9 mg). In contrast, there was a significant increase in FT4 in non-CH patients and controls after rhTSH, most pronounced with the larger dose (p <0.00?).Total T4 demonstrated as similar baseline and response pattern as FT4 (Figure 3).

### T3

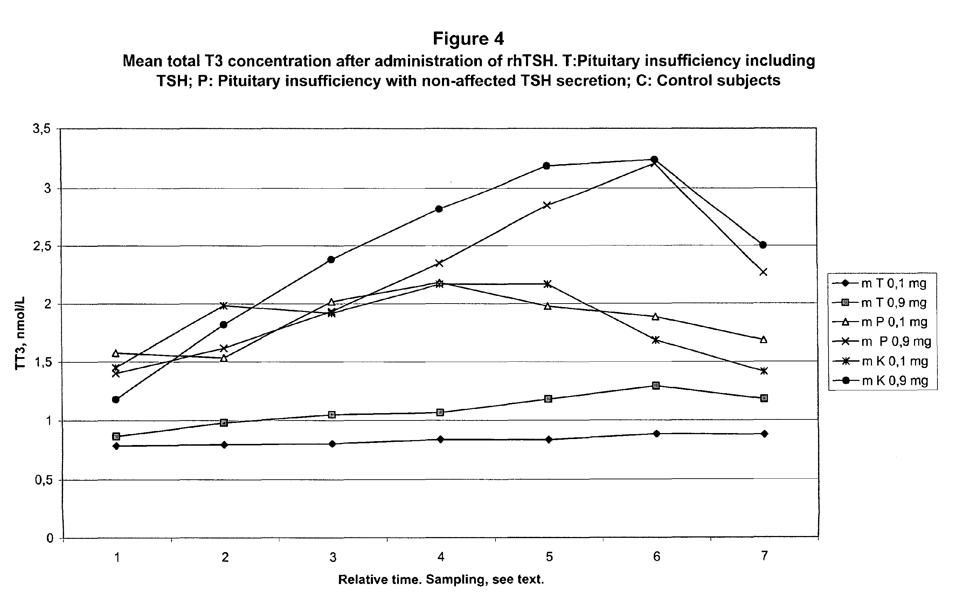

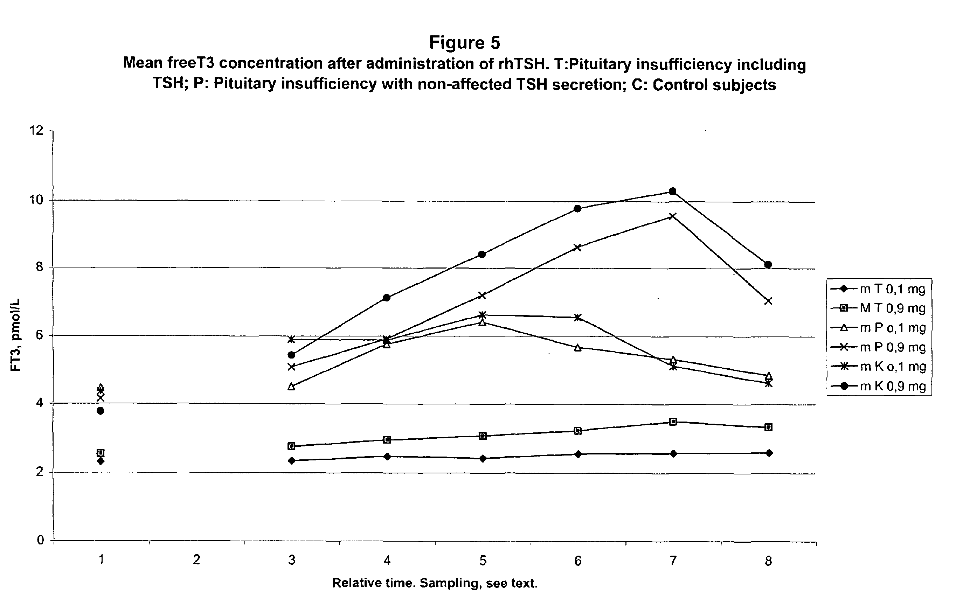

FT3 concentrations are shown in Figure 4. Before rhTSH, CH patients have significantly lower FT3 concentrations than non-CH and controls (p<0,001). After rhTSH FT3 levels were unaffected in the CH group whereas non-CH patients and controls demonstrated a significant increase in response to both dose of rhTSH (p<0.001), with a peak after 48 hours. The difference in response between doses at 48 h is significant both in non-CH and controls (p<0,001). TT3 concentration are shown in Figure 5. The observations are similar to those with FT3

### TSH

The observations were similar in all three groups, showing an increase in serum TSH with peak values after 3.5 - 7 hours, with a mea n peak concentration of serum TSH of 15 mIU/L after 0.1 mg of rhTSH and >100 mIU/L after 0.9 mg of rhTSH

## Claims

1. A method for the diagnosis of central hypothyroidism in an individual, comprising the step of:
determining the amount of thyroglobulin and thyroid hormone in a sample obtained from a patient, to which rhTSH or a pharmaceutically acceptable salt thereof has been administered, wherein an increase in thyroglobulin level and at the same time a constant thyroid hormone level is indicative that the individual has developed or suffers from central hypothyroidism.

2. The method according to claim 1, wherein the increase in thyroglobulin level is in the range of from about 50%.

3. The method according to claim 1, wherein the increase in thyroid hormones is less than 25%.

4. The method according to any of the preceding claims, wherein the rhTSh has been administered in an amount of from about 1.4 µg/kg to about 12.9 µg/kg body weight.

5. The method according to any of the preceding claims, wherein the sample has been taken during a period of from 24 to 48 hours after administration of rhTSH.

6. The method acording to any of the preceding claims, wherein the sample is a blood sample.

7. Use of recombinant human TSH (rhTSH) or a salt thereof for diagnosing/identifying central hypothyroidism.

8. The use according to claim 7, wherein the rhTSH administered stimulates thyroglobulin production in thyroid cells of an individual while at the same time thyroid hormone levels are essentially not changed.

9. The use according to claim 7, wherein the amount administered ranges from about 1.4 µg/kg to about 12.9 µg/kg body weight.

10. The use according to any of the claims 7 to 9, wherein rhTSH is administered with a pharmaceutically acceptable carrier, diluent or solvent.

11. A kit for the diagnosis of central hypothyroidism, comprising,
rhTSH or a pharmaceutically acceptable salt thereof;
means to determine the level of thyroglobulin and thyroid hormone;
and optionally
buffers and reagents used for the reactions.
